# EUROPEAN PATENT APPLICATION

(11) **EP 3 563 824 A1**
(43) Date of publication of application: **06.11.2019**
(21) Application number: 19172519.1
(22) Date of filing: 03.05.2019
(51) Int. Cl.: A61K 8/02, A61K 8/04, A61K 8/06, A61K 8/27, A61K 8/44, A61K 8/92, A61Q 15/00

(54) **ANTIPERSPIRANT FORMULATION CONTAINING NON-ALUMINUM METAL AND ESSENTIAL OIL**

(30) Priority: 04.05.2018 US 201862666973 P
(71) Applicant: Gulbrandsen Technologies, Inc., Clinton, NJ 08809 (US)
(72) Inventor: LI, Zijun, Westfield, NJ New Jersey 07090 (US)
(74) Representative: Gill Jennings & Every LLP

(57) **Abstract**

Provided is an antiperspirant formulation containing antiperspirant active ingredients, a solvent, and an additive, wherein the antiperspirant active ingredients comprise an essential oil and a non-aluminum metal, wherein the essential oil comprises mentha arvensis oil, eucalyptus globulus oil or a mixture thereof, and the non-aluminum metal comprises zinc, calcium, magnesium or a mixture thereof.

## Description

### CROSS-REFERENCE TO RELATED APPLCIATIONS

This application claims the benefit of the filing date of U.S. provisional application no. 62/666,973, filed May 4, 2018, the disclosures of which is hereby incorporated by referenced herein.

### BACKGROUND OF THE INVENTION

Currently, commercially available antiperspirant actives are mostly limited to aluminum and aluminum-zirconium metal salts, which serve the purpose of preventing, or at least limiting, perspiration on the surface of the skin.

These actives function as antiperspirants by plugging pores thereby blocking sweat release. More specifically, metal salts dissolve in sweat, diffuse into the sweat ducts where the pH of the sweat causes the salt to precipitate, thereby filling the duct and resulting in the blockage of perspiration flow.

However, antiperspirant compositions containing aluminum or aluminum-zirconium salts tend to exhibit polymerization over time, forming species with greater molecular weights. Such polymerization can be undesirable as lower molecular weight species have greater antiperspirant effect than higher molecular weight species because smaller molecules more readily and effectively plug sweat pores. Thus, maintaining low molecular weight and avoiding excessive polymerization improves the antiperspirant effect and also lowers the amount of metal salt necessary to control perspiration.

It has also been noted that metal salts as the only active ingredient, may not be fully effective. Perspiration inhibition of available commercial products, containing metal salts as the only active, range from 20-50%, depending on form and the active ingredient. Efficacy may be further limited if one decides to reduce the amount of antiperspirant used because of other concerns as further disclosed below.

In addition to concerns over the efficacy of aluminum and aluminum-zirconium salts as antiperspirant actives, questions concerning detrimental health effects from using antiperspirant containing aluminum or aluminum-zirconium salts have been raised. Because antiperspirants also function as deodorants, aluminum and aluminum-zirconium salts tend to be acidic in aqueous solution, a property which makes them effective bactericides thereby eliminating bacteria that cause odor. However, the acidic property can also be skin unfriendly, leading to some degree of skin irritation.

Moreover, others have raised the concern that the use of aluminum and aluminum-zirconium salts can result in aluminum being absorbed into the bloodstream. Additionally, use of aerosol antiperspirants containing aluminum can result in the inhalation of aluminum.

US patent publication no. 2011/0223222 to Botsaris et al. discloses a composition for topical application to inhibit or reduce perspiration and/or unpleasant body odor comprising a vehicle and a TRPM8 agonist or a plant essential oil containing a TRPM8 agonist.

Thus, there is a need for an antiperspirant formulation that does not contain aluminum or zirconium in its chemical composition and has effectiveness of an antiperspirant product over a 24 hour period as required by the FDA.

### SUMMARY OF THE INVENTION

Described herein is an antiperspirant formulation containing one or more essential oils and one or more non-aluminum metal salt. Also described herein is a personal care formulation containing one or more essential oils and a non-aluminum metal salt.

In one embodiment, the preferred essential oils are mentha arvensis oil, eucalyptus globulus oil or a mixture thereof. In a more preferred embodiment, the essential oil is a mixture of mentha arvensis oil and eucalyptus globulus oil.

In another embodiment, the preferred non-aluminum metal salt contains zinc, calcium, magnesium or a mixture thereof. In a more preferred embodiment, the non-aluminum metal salt is zinc glycinate.

In a preferred embodiment, the antiperspirant formulation does not contain aluminum or aluminum-zirconium or zirconium.

In one embodiment, one or more non-aluminum metal is included in an amount of 0.1% to 10 %, preferably 0.1% to 6%, and more preferably 0.5% to 5% based on the total weight of the antiperspirant formulation.

In one embodiment, one or more essential oils are included in an amount of 0.1% to 10 %, preferably 0.5% to 10 %, more preferably 0.5% to 5% based on the total weight of the antiperspirant formulation.

In one embodiment, the ratio of the one or more essential oils to one or more non-aluminum metal in the antiperspirant formulation is 0.1:1 to 10:1, more preferably 0.5:1 to 5:1.

In one embodiment, the antiperspirant formulation can take any form of product suited to or adapted for topical application to human skin including, without limitation, solid sticks, powders, liquids, roll-on suspensions or lotions, emulsions, gels, creams, foam, aerosols, pump and squeeze sprays, and squeeze formulations. The antiperspirant formulation can comprise a single phase or can be multi-phase system, for example a system comprising a polar phase and an oil phase, optionally in the form of a stable emulsion.

### DETAILED DESCRIPTION

The invention will be described in more detail below.

While the specification concludes with the claims particularly pointing out and distinctly claiming the invention, it is believed that the invention described herein will be better understood from the following description. All temperatures are in degrees Celsius unless specified otherwise. The invention described herein can comprise (open ended) or consist essentially of the components of the invention described herein as well as other ingredients or elements described herein. As used herein, "comprising" means the elements recited, or their equivalent in structure or function, plus any other element or elements which are not recited. The terms "having," "including," and "comprised of' are also to be construed as open ended unless the context suggests otherwise. As used herein, "consisting essentially of' means that the invention may include ingredients in addition to those recited in the claim, but only if the additional ingredients do not materially alter the basic and novel characteristics of the claimed invention. Generally, such additives may not be present at all or only in trace amounts. However, it may be possible to include up to about 10% by weight of materials that could materially alter the basic and novel characteristics of the invention as long as the utility of the compounds (as opposed to the degree of utility) is maintained. All ranges recited herein include the endpoints, including those that recite a range "between" two values. Terms such as "about," "generally," "substantially," and the like are to be construed as modifying a term or value such that it is not an absolute. Such terms will be defined by the circumstances and the terms that they modify as those terms are understood by those of skill in the art. This includes, at very least, the degree of expected experimental error, technique error and instrument error for a given technique used to measure a value.

It should be further understood that a description in range format is merely for convenience and brevity and should not be construed as an inflexible limitation on the scope of the invention. Accordingly, the description of a range should be considered to have specifically disclosed all the possible sub-ranges as well as individual numerical values within that range. For example, description of a range such as from 1 to 6 should be considered to have specifically disclosed sub-ranges such as from 1 to 3, from 1 to 4, from 1 to 5, from 2 to 4, from 2 to 6, from 3 to 6 etc., as well as individual numbers within that range, for example, 1, 2, 2.3, 3, 4, 5, 5.7 and 6. This applies regardless of the breadth of the range.

Formulations, compositions, and methods comprising an antiperspirant active that comprises a non-aluminum metal and essential oils are provided herein for inhibiting or reducing perspiration and/or unpleasant body odors by topical administration. Perspiration can occur anywhere on the body, but typically occurs in areas such as the axillae, feet, palms, back, face, neck, and groin.

The term "antiperspirant formulation" is used interchangeably with "antiperspirant composition" and "antiperspirant." The term "antiperspirant formulation" means the formulation provides antiperspirant efficacy over a 24 hour period as required by the FDA when the assessment of the antiperspirant effect was performed according to the Guidelines for Effectiveness Testing of OTC Antiperspirant Drug Products of FDA ("the Guidelines of FDA") and when the efficacy results of the investigative active was compared with the efficacy results of a standard antiperspirant containing, for example, aluminum chlorohydrate or ACH, in accordance with the hot room test provided in the Guidelines of FDA and as explained in the Examples section herein, the difference in the antiperspirant efficacy result of the investigational new active is no more than 5% or less than the antiperspirant efficacy result of the standard antiperspirant.

The methodology in the Guidelines of FDA to consisted of suspending for 17 days the use of any deodorant, ointment, cream or any other product in the region of the underarm and washing them only with the soap that was given by the researcher. After this 17-day period, the armpits were sanitized with the cleaning agent (neutral soap) for the application of the research product in one armpit, while the other armpit remained without application of any product, the participants received 4 applications controlled of the investigational product. After the application of the research product, the research participant was instructed not to wet, wash or pass any product in the armpits for the next 24 hours. After 24 hours of the last application of the investigational product the participants were submitted to heat exposure in sauna. After 60 minutes of exposure to the heat, the formulation that demonstrated the sweat reduction of 20 % or greater when compared to the sweat reduction of the armpit without application of any product is considered to have the antiperspirant formulation properties.

Even though all the test labs around the world follow the FDA guidelines to conduct a hot room test for antiperspirant actives, due to different seasons, environmental conditions, race of panelist and etc., different test labs can have different test data for the same active. Even the same lab when the test is carried out at different time, can produce the different results. Sometimes the difference can exceed 50%, so that a new active can be classified as having an antiperspirant property, while the other lab can disqualify the same active as having an antiperspirant property.

Therefore, in order to achieve reliable results, especially for new investigational active, the test results of the new investigational active must be compared with the results of the standard antiperspirant, i.e. aluminum chlorohydrate or ACH, in the same condition. For comparison, the FDA guidelines for conducting a hot room test are used, wherein the standard antiperspirant is applied to one armpit and the new investigational active is applied to the other armpit. If difference between the sweat reduction of the new active is 5% (relative) or less when compared to the sweat reduction of the standard antiperspirant containing aluminum chlorohydrate or ACH, then the new investigational active can be classified as an antiperspirant.

The term "non-aluminum metal" used herein means a metal salt which does not contain aluminum or aluminum-zirconium or zirconium in its chemical composition.

### Antiperspirant Active Ingredients

The antiperspirant active ingredients in accordance with the invention comprise both essential oils and non-aluminum metals. The inventors have found that there is a synergistic effect in the combination of essential oils and non-aluminum metals, providing an antiperspirant formulation which has antiperspirant efficacy over a 24 hour period demonstrating the sweat reduction of 20 % or greater.

The use of both a non-aluminum metal and an essential oil in an antiperspirant formulation results in a synergistic enhanced antiperspirant efficacy.

Furthermore, skin irritation is also eliminated or reduced because the antiperspirant formulation in accordance with the invention does not include aluminum and aluminum-zirconium metal salts.

In a preferred embodiment, the antiperspirant active ingredients in accordance with the invention do not include a colloidal silica solution.

### Essential Oil

The essential oils in accordance with the invention are any essential oils containing TRPM8 agonists that can activate TRPM8 channels to reduce perspiration.

Suitable essential oils include any essential oil containing TRPM8 agonists derived from plant genera or species including, without limitation, *Eucalyptus* sp. (e.g. *Eucalyptus globulus*) and *Mentha* sp. (e.g., *Mentha arvensis*), which presents eucalyptol (1,8-cineole) and menthol, respectively. Additional botanical genera and plant species whose essential oils contain a component that act as a TRPM8 agonist include, but are not limited to, *Achillea* sp. (e.g., *A. fraasii, A. holosericea, A. setacea, A. taygetea, A. teretefolia*)*, Aframomum* sp. (e.g., *A. stipulatum*)*, Artemisia* sp. (e.g., *A. haussknechtii*)*, Calamintha* sp. (e.g., *C. sylvatica*)*, Cymbopogon* sp. (e.g. *C. citratus), Eucalyptus* sp. (e.g. *E. alba, E. camadulensis, E. citriodora, E. deglupta, E. globulus, E. robusta, E. saligna, E. terticornis*)*, Lippia* sp. (e.g., *L. graveolens*), *Melalleuca* sp. (e.g., *M. leucadendron*), *Nepeta* sp. (e.g., *N. argolica, N. camphorata, N. crispa*)*, Ocimum* sp. (e.g., *O*. *americanum, O. gratissimum*), *Salvia* sp. (e.g., *S. officinalis, S. tomentosa*), *Tanacetum* sp. (e.g., *T. vulgare), Thymus* sp. (e.g., *T. lotocephalus, T. praecox, T. serpyllum, T. xmourae*), *Turnera* sp. (e.g., *T. diffusa*), as non-limiting examples of plants presenting eucalyptol (1,8-cineole); *Coriandrum* sp. (e.g. *C. sativum), Cymbopogon* sp. (e.g., *C. martini*)*, Daucus* sp. (e.g., *D. carota*), *Lonicera* sp. (e.g., *L. japonica*), *Malus* sp. (e.g., *M. pumila*)*, Melissa* sp. (e.g., *M. officinalis*), *Nepeta* sp. (e.g., *N. cataria*)*, Ocimum* sp. (e.g. *O*. *basilicum, O. gratissimum*), *Pelargonium* sp. (e.g., *P. graveolens*), *Pinus* sp. (e.g. *P. radiata*), *Polianthes* sp. (e.g., *P. tuberosa*), *Rhodiola* sp. (e.g., *R. rosea*), *Rosa* sp., *Salvia* sp. (e.g., *S. lavandulaefolia*)*, Satureja* sp. (e.g., *S. montana*), *Thea* sp. (e.g., *T. sinensis*), *Thymus* sp. (e.g., *T. vulgaris*), *Zanthoxylum* sp. (e.g. *Z. bungeanum, Z. schinifolium*)*,* as non-limiting examples of plants presenting geraniol; *Achillea* sp. (e.g., *A. millefolium*), *Actinidia* sp. (e.g., *A. macrosperma*)*, Aloysia* sp. (e.g., *A. triphylla*), *Aniba* sp. (e.g., *A. rosaeodora*), *Anthemis* sp. (e.g., *A. nobilis*), *Artemisia* sp. (e.g., *A. abrotanum, A. absinthium, A. annua, A. scoparia, A. sieberi*)*, Bursera* sp. (e.g., *B. aloexylon, B. lancifolia*)*, Cinnamomum* sp. (e.g., *C. iners, C. osmophloeum*), *Croton* sp. (e.g., *C. cajucara*), *Cymbopogon* sp. (e.g., *C. martini, C. winterianus*), *Cyperus* sp. (e.g., *C. articulatus, C. rotundus*), *Filipendula* sp. (e.g., *F. vulgaris*), *Grammosciadium* sp. (e.g., *G. platycarpum*), *Hedychium* sp. (e.g., *H. arsenic*)*. Laurus* sp. (e.g., *L. nobilis*), *Lavandula* sp. (e.g., *L. angustifolia, L. hybrida, L. officinalis*), *Lippia* sp. (e.g., *L. alba, L javanica*)*, Melissa* sp. (e.g., *M. officinalis*), *Monarda* sp. (e.g., *M. didyma*)*, Myrica* sp. (e.g., *M. gale*), *Myrtus* sp. (e.g., *M. communis*), *Ocimum* sp. (e.g., *O*. *basilicum, O. sanctum*), *Origanum* sp. (e.g., *O*. *vulgare*), *Plantago* sp. (e.g., *P. asiatica*), *Pseudopanax* sp., *Sassafras* sp. (e.g., *S. albidum*)*, Satureja* sp. (e.g., *S. parnassica*)*, Strychnos* sp. (e.g., *S. spinosa*), *Thymbra* sp. (e.g., *T. capitata*)*, Thymus* sp. (e.g., *T. albicans, T. carnosus, T. mastichina, T. vulgaris*), *Zanthoxylum* sp. (e.g., *Z. armatum, Z. bungeanum, Z. rhoifolium, Z. schinifolium*), *Zataria* sp. (e.g., *Z. multiflora*), as non-limiting examples of plants presenting linalool; *Angelica* sp. (e.g., *A. glauca*)*, Baccharis* sp. (e.g., *B. dracunculifolia*)*, Comptonia* sp. (e.g., *C. peregrina*)*, Discaria* sp. (e.g., *D*. *americana*), *Echinodorus* sp. (e.g., *E. grandiflorus*), *Eryngium* sp. (e.g., *E. comiculatum*)*, Melaleuca* sp., *Piper* sp. (e.g., *P. gaudichaudianum*), *Pittosporum* sp. (e.g., *P. tobira*)*, Pseudopanax* sp., *Stachys* sp., *Strychnos* sp. (e.g., *S. spinosa*), *Thymus* sp. (e.g., *T. serpyllum*)*, Virola* sp. (e.g., *V. surinamensis*)*, Zanthoxylum* sp. (e.g., *Z. hyemale, Z. rhoifolium, Z. setulosum*)*,* as non-limiting examples of plants presenting nerolidol; *Abies* sp. (e.g., *A. coreana*)*, Achillea* sp. (e.g., *A. millefolium*)*, Aframomum* sp. (e.g., *A. danieffi, A. stipulatum*)*, Alpinia* sp. (e.g., *A. galanga*)*, Artemisia* sp. (e.g., *A. absinthium, A. feddei, A. haussknechtii, A. lavandulaefolia*)*, Bursera* sp., *Cinnamomum* sp. (e.g., *C. osmophleum, C. zeylanicum*)*, Citrus* sp., *Cryptomeria* sp. (e.g., *C. japonica*), *Cymbopogon* sp. (e.g., *C. citratus*), *Discaria* sp. (e.g., *D. americana*), *Dracocephalum* sp. (e.g., *D. kotschyi*), *Drimys* sp. (e.g., *D. granatensis*), *Eucalyptus* sp. (e.g., *E. alba, E. camadulensis, E. citriodora, E. deglupta, E. globulus, E. propinqua, E. robusta, E. saligna, E. terticornis, E. urophylla*)*, Eugenia* sp. (e.g., *E. dysenterica*), *Heteropyxis* sp. (e.g., *H. dehniae*), *Houttuynia* sp. (e.g., *H. cordata*)*, Juniperus* sp. (e.g., *J*. *procera*)*, Laurus* sp. (e.g., *L. nobilis*)*, Lavandula* sp. (e.g., *L. angustifolia, L. viridis*), *Lippia* sp. (e.g., *L. multiflora*), *Majorana* sp. (e.g., *M. hortensis*), *Melaleuca* sp. (e.g., *M. alternifolia*)*, Myrica* sp. (e.g., *M. gale*), *Ocimum* sp. (e.g., *O*. *americanum, O*. *gratissimum*), *Origanum* sp. (e.g., *O*. *cordifolium*), *Pelargonium* sp., *Peucedanum* sp. (e.g., *P. ostruthium*), *Pimenta* sp. (e.g., *P. racemosa*)*, Pistacia* sp., *Rhododendron* sp. (e.g., *R. tomentosum*)*, Salvia* sp. (e.g., *S. lavandulaefolia, S. sclarea*)*, Shorea* sp. (e.g., *S. robusta*)*, Tanacetum* sp. (e.g., *T. vulgare*), *Tetradenia* sp. (e.g., *T. riparia*)*, Thymus* sp. (e.g., *T. caespititius, T. kotschyanus, T. persicus, T. vulgaris*), *Zanthoxylum* sp. (e.g., *Z. bungeanum, Z. schinifolium*), *Zea* sp. (e.g., *Z. mays), Zingiber* sp. (e.g., *Z. roseum*), as non-limiting examples of plants presenting terpineol; *Aloysia* sp. (e.g., *A. polystachya*)*, Anethum* sp. (e.g., *A. graveolens*), *Artemisia* sp. (e.g., *A. alba*), *Balsamita* sp. (e.g., *B. major, B. suaveolens*), *Carum* sp. (e.g., *C. carvi*), *Dracocephalum* sp. (e.g., *D. grandiflorum, D. renati, D. ruyschiana*)*, Lippia* sp. (e.g., *L. alba, L. javanica*)*, Mentha* sp. (e.g, *M. piperita, M. spicata, M. suaveolens*), *Nigella* sp. (e.g., *N. sativa*), *Ocimum* sp. (e.g., *O*. *basilicum*), *Origanum* sp. (e.g., *O*. *majorana*)*,* as non-limiting examples of plants presenting carvone; *Achyrocline* sp. (e.g., *A. satureioides*)*, Lavandula* sp. (e.g., *L. stoechas*), *Magnolia* sp. (e.g., *M. biondii, M. denudata, M. sprengeri*), *Mentha* sp. (e.g., *M. aplocalyx, M. aquatica, M. citrata, M. crispa, M. gentilis, M. longifolia, M. longifolia, M. piperita, M. rotundifolia, M. suaveolens*)*, Minthostachys* sp. (e.g., *M. verticillata*)*, Perilla* sp. (e.g., *P. frutescens*)*, Schizonepeta* sp. (e.g., *S. tenuifolia*)*,* as non-limiting examples of plants presenting menthol; *Bystropogon* sp. (e.g., *B. canariensis, B. origanifolius, B. plumosus, B. wildpretii*)*, Cunila* sp. (e.g., *C. polyantha*)*, Lavandula* sp. (e.g., *L. stoechas*), *Melissa* sp. (e.g., *M. officinalis*), *Mentha* sp. (*M. aplocalyx, M. aquatica, M. arvensis, M. crispa, M. gentilis, M. longifolia, M. piperita, M. pulegium, M. rotundifolia, M. sachalinensis, M. suaveolens*)*, Minthostachys* sp. (e.g., *M. mollis, M. verticillata*)*, Pelargonium* sp. (e.g., *P. tomentosum*)*,* as non-limiting examples of plants presenting menthone; *Cunila* sp. (e.g., C. *polyantha*)*, Lavandula* sp. (e.g., *L. stoechas*)*, Mentha* sp. (e.g, *M. arvensis, M. cervina, M. crispa, M. microphylla, M. piperita, M. pulegium, M. spicata, M. suaveolens*)*, Micromeria* sp. (e.g., *M. cilicica*)*, Minthostachys* sp. (e.g., *M. mollis, M. verticillata*)*, Origanum* sp. (e.g., *O*. *majorana*)*, Poliomintha* sp. (e.g. *P. incana*)*, Salvia* sp. (e.g., *S. rhytidea*)*, Schizonepeta* sp. (e.g., *S. tenuifolia*)*, Thevetia* sp. (e.g., *T. peruviana*)*, Ziziphora* sp. (e.g., *Z. clinopodioides, Z. persica, Z. taurica*)*,* as non-limiting examples of plants presenting pulegone; *Arnica* sp. (e.g., *A. longifolia*)*, Aster* sp. (e.g, *A. hesperius*), *Carum* sp. (e.g., *C. copticum*), *Coridothymus* sp. (e.g., *C. capitatus*). *Lantana* sp. (e.g., *L. achyranthifolia*)*, Lavandula* sp. (e.g., *L. multifida*)*, Lippia* sp. (e.g., *L. graveolens, L. multiflora, L. sidoides*)*, Majorana* sp. (e.g., *M. syriaca*)*, Micromeria* sp. (e.g., *M. nervosa*), *Monarda* sp. (e.g., *M. didyma*)*, Mosla* sp. (e.g., *M. chinensis*), *Nigella* sp. (e.g., *N. sativa*), *Origanum* sp. (e.g., *O*. *acutidens, O. compactum, O. cordifolium, O. minutiflorum, O. onites, O. sativum, O. virens, O. vulgare*), *Plectranthus* sp. (e.g., *P. cylindraceus*), *Satureja* sp. (e.g., *S. cuneifolia, S. hortensis, S. khuzistanica, S. laxiflora, S. montana, S. parnassica, S. thymbra, S. wiedemanniana*)*, Stachys* sp. (e.g., *S. persica*)*, Thymbra* sp. (e.g., *T. capitata, T. spicata*)*, Thymus* sp. (e.g., *T. caespititius, T. eigii, T. kotschyanus, T. longicaulis, T. pectinatus, T. persicus, T. pulegioides, T. revolutus, T. serpyllum, T. vulgaris, T. zygioides*), *Zataria* sp. (e.g., *Z. multiflora*), as non-limiting examples of plants presenting carvacrol. (See, for example, Behrendt et al., (2004) British Journal of Pharmacology 141: 737-745; Sonboli et al., (2006) Z. Naturforsch 61:160-164; Unlü et al., (2002) J. Ethnopharmacol. 83:117-121; Salgueiro et al., (2000) Biochem. Syst. Ecol. 28:457-470; Jalali Heravi et al. (2007) J. Chromatogr. A. 1160:81-89; Chiang et al., (2005) Clin. Exp. Pharmacol. Physiol. 32:811-816; Thompson et al., (2003) J. Chem. E-col. 29:859-880; Beytia et al., (1969) Arch. Biochem. Biophys. 129:346-356; Radulović et al., (2007) Fitoterapia 78:565-570; Jia et al. (1999) Arch. Biochem. Biophys. 372:143-149; Shafiee and Javidnia (1997) Planta Med. 63:371-372; Klopell et al. (2007), Z Naturforsch 62:537-542; Hoet et al. (2006) Planta Med. 72:480-482; Simionatto et al. (2005) Planta Med. 71:759-763, Cha et al, (2007) J Microbiol Biotechnol. 17:2061-2065; Jalali and Sereshti (2007) J Chromatogr A. 1160:81-89; Prakash et al. (2006) J Ethnopharmacol. 106:344-347; Lota et al. (2002) J Agric Food Chem. 50:796-805; Goren et al. (2002) Z Naturforsch 57:797-800, contents of which are incorporated hereinto by referencesdfd

In one embodiment, one or more essential oils may be used in the antiperspirant formulation as the active ingredient.

In a preferred embodiment, the essential oils are a mixture of Mentha arvensis oil and Eucalyptus globulus oil.

In one embodiment, one or more essential oils are included in an amount of 1% to 10%, more preferably 2 to 6% based on the total weight of the antiperspirant formulation.

### Non-Aluminum Metal

The non-aluminum metal in accordance with the invention is any metal salt which does not contain aluminum or aluminum-zirconium or zirconium in its chemical composition. In one embodiment, the preferred non-aluminum metal salt contains zinc, calcium, or magnesium. In a more preferred embodiment, the non-aluminum metal salt are zinc glycinate, calcium glycinate, and magnesium glycinate. In a most preferred embodiment, the non-aluminum metal is zinc glycinate. In one embodiment, the non-aluminum metal salt contains alkaline and alkaline earth glycinate Examples of alkaline glycinate include, without limitation, lithium, sodium, potassium, ribudium, caesium and francium glycinates. Examples of alkaline earth glycinate include, without limitation, beryllium, magnesium, calcium, strontium, barium and radium.

In one embodiment, one or more non-aluminum metals may be used in the antiperspirant formulation as the active ingredient.

In one embodiment, one or more non-aluminum metals are included in an amount of 1% to 10%, more preferably 2 to 6% based on the total weight of the antiperspirant formulation.

### Solvent

In one embodiment, a solvent in accordance with the invention includes, without limitation, water, polypropylene glycol, polyethylene glycol, ethanol, glycerol, ethylene glycol, 1,2,4-butanetriol, 1,2,6-hexanetriol, ethanol, isopropanol, butanetriol, sorbitol esters, butanediol, butylene glycol, hexylene glycol, methylpropanediol, pyrrolidone, N-methylpyrrolidone, dimethyl sulfoxide, dimethyl sulfone and mixtures thereof.

### Additives

Other additives or optional ingredients that can be included in an antiperspirant formulation include emulsifiers, skin care additives, perfumes, preservatives, colorants, watersoluble alcohols such as C₂₋₈ alcohols including ethanol, glycols including propylene glycol, dipropylene glycol, tripropylene glycol and mixtures thereof; glycerides including mono-, di- and triglycerides; organic acids, alcohols and esters; surfactants including emulsifying and dispersing agents, amino acids including glycine; surfactants including thickeners and gelling agents, for example polymers, silicates and silicon dioxide; emollients.

In one embodiment, the antiperspirant formulation can take any form of product suited to or adapted for topical application to human skin including, without limitation, solid sticks, powders, liquids, roll-on suspensions or lotions, emulsions, gels, creams, foam, aerosols, pump and squeeze sprays, and squeeze formulations. The antiperspirant formulation can comprise a single phase or can be multi-phase system, for example a system comprising a polar phase and an oil phase, optionally in the form of a stable emulsion.

### Personal Care Composition

Another aspect of the invention is directed to a personal care composition containing both essential oils and non-aluminum metal and at least one dermatologically or cosmetically acceptable carrier.

As used herein the term "carrier" will be understood by one of ordinary skill in the art to mean any component that can be used as a delivery vehicle to facilitate application and prolonged contact of the essential oils and non-aluminum metal to the skin, and which are inert and non-toxic to and compatible and with the skin.

Water absorbing agents that are known for cosmetic applications and are usually porous, composed of micro channels resulting in a high absorption capacity and the ability to absorb a different variety of materials could be included in the personal care formulation. Such compounds may have affinity to hydrophilic or lipophilic molecules. In some instances, it is preferable to combine multiple water absorbing agents in order to maximize absorption capability. Suitable water absorbing agents include, without limitation, silica, cotton powder, bamboo silk, and zeolites.

Emollients, in solid or liquid form, provide an increase in skin lubricity and may be included for a more aesthetically pleasing product as it can reduce whitening on the skin. The composition may contain emollients in any desired amount to achieve the desired emollient effect.

Non-volatile emollients are preferable. Classes of non-volatile emollients include non-silicone and silicone emollients. Non-volatile, non-silicone emollients include C₁₂₋₁₅ alkyl benzoate. The non-volatile silicone material can be a polyethersiloxane, polyalkyarylsiloxane or polyethersiloxane copolymer. Examples include, but are not limited to, PPG-14 butyl ether, PPG-15 stearyl ether, PPG-3 myristyl ether, stearyl alcohol, stearic acid, glyceryl monoricinoleate, isobutyl palmitate, glyceryl monostearate, isocetyl stearate, sulphated tallow, oleyl alcohol, propylene glycol, isopropyl laurate, mink oil, sorbitan stearate, cetyl alcohol, hydrogenated castor oil, stearyl stearate, hydrogenated soy glycerides, isopropyl isostearate, hexyl laurate, dimethyl brassylate, decyl oleate, diisopropyl adipate, n-dibutyl sebacate, diisopropyl sebacate, 2-ethyl hexyl palmitate, isononyl isononanoate, isodecyl isononanoate, isotridecyl isononanoate, 2-ethyl hexyl palmitate, 2-ethyl hexyl stearate, Di-(2-ethyl hexyl) adipate), Di-(2-ethyl hexyl) succinate, isopropyl myristate, isopropyl palmitate, isopropyl stearate, octacosanol, butyl stearate, glyceryl monostearate, polyethylene glycols, oleic acid, triethylene glycol, lanolin, castor oil, acetylated lanolin alcohols, acetylated lanolin, petrolatum, isopropyl ester of lanolin, fatty acids, mineral oils, butyl myristate, isostearic acid, palmitic acid, PEG-23 oleyl ether, olelyl oleate, isopropyl linoleate, cetyl lactate, lauryl lactate, myristyl lactate, quaternised hydroxy alkyl, aminogluconate, vegetable oils, isodecyl oleate, isostearyl neopentanoate, myristyl myristate, oleyl ethoxy myristate, diglycol stearate, ethylene glycol monostearate, myristyl stearate, isopropyl lanolate, paraffin waxes, glycyrrhizic acid, hydrocyethyl stearate amide.

In one embodiment, the emollient is selected from linear silicones, cyclic silicones, hydrocarbons, polyhydroxy alcohols having more than 3 carbon atoms, liquid or solid polyalkyleneglycol ethers containing a polypropylene glycol (PPG) moiety and terminating in an alkyl ether, and combinations thereof. In another embodiment, the emollient is a volatile silicone having a flash point of 100° C. or less, such as cyclomethicone or trisiloxane. In another embodiment, the emollient is a nonvolatile silicone, such as dimethiconol or a longer chain dimethicone.

Surfactants and emulsifiers, as noted above, may be included in the composition. Emulsifiers useful in the composition include nonionic, anionic, cationic, amphoteric or zwitterionic and blends thereof. Examples are, without limitation, polyethylene glycol 20, sorbitan monolaurate (Polysorbate 20), polyethylene glycol 20 stearyl ether (Brij 78, Steareth 20), polyethylene glycol ether of lauryl alcohol (Laureth 23), polysorbate 80 (Tween 80), and lecithin.

Film formers may also be included in the composition. For example, water-insoluble films of polymers create occlusion on the skin, thereby reducing wetness. Such films are a barrier to the passage of sweat. Polymers used should be occlusive, insoluble in water, and high in degree of adhesion, such as acrylates/octylacrylamide copolymers.

The composition may further comprise of preservatives. Preservatives are required to prevent product damage caused by bacteria, yeast or mold, and to protect the product from inadvertent contamination by the consumer during use. Useful preservatives include sodium benzoate, ethylhexylglycerin and caprylyl glycol, benzyl alcohol, methyl paraben, propyl paraben, DMDM hydantoin, methylchloroisothiaoline, methylisothiazolinone, imidazolidinyl urea phenoxyethanol, sodium benzoate and benzoic acid. EDTA and salts thereof are often used to further enhance preservation.

The composition may further comprise of antioxidants used to prevent oxidation. Useful antioxidants include ascorbic acid, tocopheryl acetate and polypnehols. In another embodiment, tocopheryl acetate (stabilized vitamin E) may be used as anti-oxidant agent.

The composition may further comprise linear chain alcohols, amines, ether-alcohols, cetyl alcohol, stearyl alcohol and cetearyl alcohol, classified as emulsion stabilizing agents.

The composition may further comprise of polyethylene beads. The polyethylene component contributes to the reduction in synersis and also provides a powdery feel to the products. The polyethylene beads may have a density in the range of 0.91-0.98 g/cm³ and a particle size in the range of 5-40 microns, with one particular type of polyethylene having a particle size of 20 microns. Several types of suitable polyethylene beads that are commercially available are MICROTHENE FN 510 from Equistar Chemicals LP (Houston, Tex.); and ACUMIST A-6 from Allied Signal Corp., Morristown, N.J.).

In another embodiment, the composition may further comprise additional cooling agents such as, but not limited to, L-menthol; menthyl lactate; menthone glycerine; menthone glycerin acetal; (-)-isopulegol, N-ethyl-5-methyl-2-(1-methylethyl)-cyclohexanecarboxamide; N-ethyl-p-menthane-3-carboxzamide; 4-methyl-3-(1-pyrrolidinyl)-2[5H]-furanone; N,2,3-trimethyl-2-isopropylbutanamide (also known as 2-isopropyl-N,2,3-trimethylbutyramide); menthoxypropanediol; methanediol; vanillyl butyl ether; in combination with a selected superabsorbent material thereby providing a product that balances a cooling effect with a dry sensation over an extended period of time. In a further embodiment, the composition is surfactant-free and anhydrous. In a further embodiment, the composition is made in a form of soft solids.

In most embodiments, the composition may further include other functional ingredients, which provide benefits to one's body. Such materials are in general well-known to a person skilled in the art of personal care composition, and may include moisturizing agents, antibacterial agents, antimicrobial agents, and perfumes. If desired, deodorant agents can be included, for example an odor reducing agent such as a sulfur precipitating agent, e.g., copper gluconate, zinc gluconate, zinc citrate.

Examples of moisturizing agents are, but not limited to, allantoin, glycerol, polyglycerylmethacrylate, panthenol, polyols, ceramide, borage oil (linoleic acid), hyaluronic acid, sodium peroxylinecarbolic acid (sodium PCA), wheat protein (e.g., laurdimonium hydroxypropyl hydrolyzed wheat protein), hair keratin amino acids, panthenol; primrose oil; GLA 3 and other fish oils that may include, for example, the omega-3 and omega-6 oils and/or linoleic acid; and flax seed oil, and mixtures thereof. Other moisturizing agents can also be used.

Other optional ingredients include ingredients conventionally employed in antiperspirant, deodorant, or cosmetic products. For example, propellants commonly used in aerosol compositions herein comprise hydrocarbons (or much less desirably halohydrocarbons) having a boiling point of below 10° C. and especially those with a boiling point below 0° C. It is more preferable to employ liquefied hydrocarbon gases, and especially C3 to C6 hydrocarbons, including propane, isopropane, butane, isobutane, pentane and isopentane and mixtures of two or more thereof. Preferred propellants are isobutane, isobutane/isopropane, isobutane/propane and mixtures of isopropane, isobutane and butane. Other propellants that can be considered include alkyl ethers, such as dimethyl ether or compressed non-reactive gasses such air, nitrogen or carbon dioxide.

In another embodiment, the composition can take any form of a cosmetic or pharmaceutical product suited to or adapted for topical application to human skin including, without limitation, solid sticks, powders, liquids, roll-on suspensions or lotions, emulsions, gels, creams, foam, aerosols, pump and squeeze sprays, and squeeze formulations. The composition can comprise a single phase or can be multi-phase system, for example a system comprising a polar phase and an oil phase, optionally in the form of a stable emulsion.

In the form of a solid or semi-solid or stick, the composition further comprises solidifying agent, wherein the composition is in the form of a solid or semi-solid at ambient temperature (e.g., 25°C and below). Accordingly, the composition may comprise, for example, mixtures of waxes and oils and solutions based on aqueous, propylene glycol and/or alcohol mixtures solidified for example by sodium stearate. In a preferred embodiment, the solidifying agent is selected from the group consisting of a wax and sodium stearate.

In another embodiment, compositions in a solid state may also be finely divided and used in the form of powders.

Majority of antiperspirant solids use stearyl alcohol as a gelling agent. Emollients are often added to impart a softer feel and glideability. Most antiperspirant solids also contain talc and/or silica, which is effective as a suspending agent keeping the AP active homogenously suspended throughout the solid. Other ingredient can include, without limitation, coloring agents, titanium dioxide as an opacifying agent, and allantoin as a soothing agent.

A liquid form of the composition may be directly utilized in oil-in water and water-in oil emulsions, and formulated as roll-on products.

In a further embodiment, the liquid-based roll-on is usually an oil-in-water emulsion rather than a water-in-oil type due to the poorer efficiency of the latter. The oil-in-water emulsion presents the active ingredient in a readily accessible solution form in the external phase. Because the active ingredient ends up in the dissolved state, one can use either a liquid or solid AP active.

In a further embodiment, the composition may also be a clear water-in-oil roll-on. These compositions are relatively new on the market. They demonstrate superior aesthetics and leave no residue or deposit on the skin after application.

Gel is a colloid in which the disperse phase has combined with the continuous phase to produce a viscous, jelly-like product. Gels can be aqueous or nonaqueous. Gels will typically comprise a vehicle comprising a gelling agent and a solvent. A silicone surfactant may also be included to achieve stability.

Emulsions, such as lotions and creams, can be the oil-in-water type or water-in-oil type. Triphase emulsions such as water-in-oil-in-water type may also be used. Oils useful in both types of emulsions, and also for solvents in solvent-based vehicles in general, include hydrocarbon oils and waxes (e.g., petrolatum, mineral oil, micro-crystalline waxes, polyalkenes, paraffins, cerasin, ozokerite, polyethylene, perhydrosqualene, polyalphaolefins, hydrogenated polyisobutenes and combinations thereof). Preferred are fatty acid derivatives, cholesterol, cholesterol derivatives, diglycerides and triglycerides (e.g., castor oil, soy bean oil, derivatized soybean oils such as maleated soy bean oil, safflower oil, cotton seed oil, corn oil, walnut oil, peanut oil, olive oil, cod liver oil, almond oil, avocado oil, palm oil, sesame oil, vegetable oils and vegetable oil derivatives, sunflower seed oil, coconut oil and derivatized coconut oil, cottonseed oil and derivatized cottonseed oil, jojoba oil, cocoa butter and combinations thereof, as well as any of the aforementioned oils that have been partially or fully hydrogenated), acetoglyceride esters (e.g., acetylated monoglycerides), alkyl esters, alkenyl esters (e.g., oleyl myristate, oleyl stearate, oleyl oleate, and combinations thereof), lanolin and its derivatives (e.g., lanolin, lanolin oil, lanolin wax, lanolin alcohols, lanolin fatty acids, isopropyl lanolate, acetylated lanolin, acetylated lanolin alcohols, lanolin alcohol linoleate, lanolin alcohol ricinoleate, hydroxylated lanolin, hydrogenated lanolin and combinations thereof), wax esters (e.g., beeswax and beeswax derivatives, spermaceti, myristyl myristate, stearyl stearate and combinations thereof), sterols and phospholipids, and combinations thereof. Examples of alkyl esters include isopropyl esters of fatty acids and long chain esters of long chain fatty acids, e.g., SEFA (sucrose esters of fatty acids), pentaerythritol esters, aromatic mono, di or triesters, cetyl ricinoleate, isopropyl palmitate, isopropyl myristate, cetyl ricinoleate and stearyl ricinoleate. Other examples include hexyl laurate, isohexyl laurate, isohexyl palmitate, decyl oleate, isodecyl oleate, hexadecyl stearate, decyl stearate, isopropyl isostearate, diisopropyl adipate, diisohexyl adipate, dihexyldecyl adipate, diisopropyl sebacate, acyl isononanoate lauryl lactate, myristyl lactate, cetyl lactate, and combinations thereof. Still other suitable oils include milk triglycerides (e.g., hydroxylated milk glyceride) and polyol fatty acid polyesters. Also useful are vegetable waxes such as carnauba and candelilla waxes; sterols such as cholesterol, cholesterol fatty acid esters; and phospholipids such as lecithin and derivatives, sphingo lipids, ceramides, glycosphingo lipids, and combinations thereof.

When the composition is in the form of foam, liquid vehicles described above may contain dispersions of gas in the liquid phase. The gas globules may be of any size, from colloidal to macroscopic, like soap bubbles. Typical liquid foams are those used in shaving creams, etc.

In another embodiment, the composition can be for, but not limited to, an antiperspirant and/or deodorant, facial wash, body wash, a shower gel, a bar soap, a hand soap, a shampoo, a hair conditioner, a toothpaste, a dentifrice, or a mouth wash. The composition can be provided in any suitable container including, without limitation, an aerosol can, tube or container with a porous cap, roll-on container, bottle, container with an open end, etc.

In yet another embodiment, a personal care product comprising the personal care composition is provided, wherein the personal care product is a moisturizer, a cream, a lotion, a skin softener, a foundation, a night cream, a lipstick, a cleanser, a toner, a sunscreen, a cosmetic mask, a shampoo, a conditioner, an insect repellent, an anti-aging product, a body and hand preparation, a skin care preparation, a face and neck preparation or cosmetics.

### EXAMPLES

### Example 1 - Roll On Formulation

The antiperspirant roll on formulation was manufactured using eucalyptus oil (1.5 w/w %), menthe avernesis oil (1.5 w/w %) and zinc glycinate (1.5 w/w %) as the antiperspirant active ingredients. The entire list of ingredients for this formulation of Example 1 is identified below in the table.

| Ing. No. | Ingredients | EOZG-3 |
|---|---|---|
| | | % w/w |
| 1 | Water | 55.35 |
| 2 | Zinc glycinate 5 % solution (which is about 1.5% w/w zinc glycinate) | 30.00 |
| 3 | Steareth-21 | 04.00 |
| 4 | C12-15 alkyl benzoate | 03.00 |
| 5 | Propylene glycol | 03.00 |
| 6 | Mentha arvensis oil | 01.50 |
| 7 | Eucalyptus globulus oil | 01.50 |
| 8 | Acrylates/c10-30 alkyl acrylate crosspolymer | 00.50 |
| 9 | Phenoxyethanol | 00.30 |
| 10 | Sodium hydroxide 20 % solution | 00.80 |
| 11 | Ethylenediaminetetraacetic Acid | 00.05 |

First, acrylates/c10-30 alkyl acrylate crosspolymer was slowly added into water under stirring and it was stirred for 30-40 minutes. After obtaining a lump free dispersion, sodium hydroxide 20% solution was added under stirring to make a gel formulation in a vessel. The gel was heated at 70°C. In another vessel, steareth-21 and c12-15 alkyl benzoate were mixed and melted and the mixture was heated to 70°C. At 70°C, the mixture of steareth-21 and c12-15 alkyl benzoate was added into the gel formulation. The mixture was homogenized at the temperature of between 60°C - 70°C and the roll-on base material was prepared. In a separate vessel, propylene glycol, mentha arvensis oil, eucalyptus globulus oil and ethylenediaminetetraacetic acid were heated at 55-60°C. Then, this mixture was added to the prepared roll-on base material at 55-60°C. Then, phenoxyethanol was added at a temperature below 55°C and it was stirred well. Then, zinc glycinate 5 % solution (∼1.5% ZnGly) was added at a temperature below 50°C under low stirring. The mixture was then cooled with stirring at normal room temperature to obtain a roll-on antiperspirant formulation. After 48 hours of production of the roll-on antiperspirant formulation, quality and stability tests were performed as shown below.

### Quality parameters

| Parameters | EOZG-3 |
|---|---|
| Appearance | Air free liquid preparation |
| Color | Milky white |
| Odour | Pungent essential oils |
| pH 10 % solution | 7.5-8.5 |
| Viscosity cps (Brookfield viscometer, Sp No 64, rpm 20) | 1000- 5000 cps |
| Centrifuge test (3000rpm, 20 minutes at 45C) | Pass, Stable |
| Stability test at 40°C, 3 months | Pass, Stable |

### Hot Room Study

The assessment of the antiperspirant effect was performed according to the Guidelines for Effectiveness Testing of OTC Antiperspirant Drug Products do Food and Drug Administration (FDA). The methodology consisted of suspending for 17 days the use of any deodorant, ointment, cream or any other product in the region of the underarm and washing them only with the soap that was given by the researcher.

After this 17-day period, the armpits were sanitized with the cleaning agent (neutral soap) for the application of the research product in one armpit, while the other armpit remained without application of any product, the participants received 4 applications of the investigational product. The investigational product was applied to a 100 cm² area in the underarms. The investigational product application procedure was repeated four days consecutively. 0.4 ±0.1g of the investigational product was applied in one of the armpits, previously randomized. The investigational product was spread evenly on the underarm until completely cover the bounded area.

After 24 hours of last application of the investigational product, the participants were submitted to the exposure to heat in the sauna.

Research participants remained in the sauna with a temperature of 37.8 ± 2°C and relative air humidity = 35 ± 5%, for a total period of 80 minutes.

To collect sweating were used of silica gel pads or absorbents, stored in polyethylene containers closed.

The first pads were placed in the armpits of the participants immediately after they entered the sauna and remained in the armpits for 40 minutes. After 40 minutes, the pads were removed and discarded. Then, new pre-weighed pad was placed, being replaced every 20 minutes. The pads relative to the times of 60 to 80 minutes of exposure were removed and weighed.

For comparative evaluation of the effect of the investigational product and control was applied the Wilcoxon Signed Rank test, with a 95% confidence level.

According to the study protocol and procedures used to evaluate the antiperspirant effect 24 hours after the application of the Example 1 formulation, the following is the conclusion:
- After 60 minutes of exposure to heat, the investigational product (Example 1) reduced 47.8% of sweating. 100.0% of the participants had reduced sweating greater than 20%. 100.0% of the participants had reduced sweating greater than 30%.
- After 80 minutes of exposure to heat, the investigational product (Example 1) reduced 43.4% of sweating. 100.0% of the participants had reduced sweating greater than 20%. 100.0% of the participants had reduced sweating greater than 30%.

### Antiperspirant Effectiveness Data

| Number of Applications | Panelists | 24 Hours after the application of Example 1 (EOZG3) | % Sweat reduction |
|---|---|---|---|
| 4 | 30 (all females) | 60 minutes exposure of heat | 47.8% |
| | | 80 minutes exposure of heat | 43.4% |

### Example 2 - Parallel Hot Room Study

In order to compare the efficacy of inventive formulation containing both essential oils and non-aluminum metal containing zinc (EOZG3) with standard antiperspirant, i.e. aluminum chlorohydrate (ACH), both EOZG3 and ACH were formulated into stick formulations and parallel hot room study was conducted.

| Phase | Ingredients | % W/W ACH Powder (D 1097) | % W/W Essential Oil & Zinc Glycinate (EOZG3) |
|---|---|---|---|
| **A** | Behenyl alcohol | 15.00 | 16.00 |
| | Hydrogenated castor oil | 03.20 | 03.50 |
| | Polyethylene | 05.00 | 05.50 |
| | | | |
| **B** | Cyclomethicone | 42.03 | 46.50 |
| | C12-15 alkyl benzoate | 03.00 | 03.00 |
| | Isopropyl myristate | 08.00 | 08.00 |
| | Ppg-15 stearyl ether | 09.00 | 09.00 |
| | | | |
| **C** | Silica | 01.25 | 01.50 |
| | Talc | 01.25 | 02.50 |
| | Zinc glycinate | 00.00 | 01.50 |
| | | | |
| **D** | Essential oils (Mentha arvensis oil+Eucalyptus oil) | 0.00 | 03.00 |
| | D1097 (∼ 10% anhydrous) | 12.27 | 0 |

To produce respective stick formulations, first, the phase A ingredients were mixed and heated at a temperature of 80-85°C. After a clear mixture was obtained, a mixture of the phase B ingredents were added to the mixture of phase A ingredients at a temperature of 75-78°C. Next, a blend of phase C ingredients was added at a temperature of 75-78°C and was stirred well. Thereafter, the phase D ingredients were added at a temperature of 65-70°C under stirring and the hot mixtures were filled in stick containers. The mixtures were cooled down and after 24 hours of production of the stick formulations, quality and stability tests were performed as shown below.

### Quality Parameters

| | ACH Powder (D1097) | Essential Oil & Zinc Glycinate (EOZG3) |
|---|---|---|
| Appearance | Solid stick | Solid stick |
| Color | Off white | Off white |
| Odor | Characteristics | Characteristics |
| Freezing point | 63-68°C | 63-68°C |
| Softening point | 50-55°C | 50-55°C |

### Hot Room Study

The methodology consisted of suspending use of use of any deodorant, ointment, cream or any other product in the region of the underarm, beyond those supplied by the laboratory for a period of 24 days. During the conditioning period, the participants were instructed to wash their underarms only with a mild soap dispensed by the laboratory. After the conditioning period, it was carried out the initial assessment in sauna to check the amount of sweat produced per axilla without application of any products to obtain baseline sweat collection.

To be approved, it is necessary that the participant produced at least 100mg of sweating per axilla for two consecutive sessions (T1 and T2) of 20 minutes. The approved participants received four applications of the investigational product EOZG3 stick formulation in one armpit and in the other, four applications of the stick formulation containing ACH powder. Posteriorly it was performed the sauna of 48 hours after the four controlled applications.

According to the study protocol and procedures used to evaluate the antiperspirant effect after four controlled application, the following is the conclusion:
- The EOZG3 presented a reduction of 50.7% of sweating compared to the baseline condition.
- The D1097 presented a reduction of 49.2% of sweating compared to the baseline condition.
- The P value obtained was <0.0001.

This parallel hot room study clearly indicate EOZG3 is an effective antiperspirant and performed superior than that of the standard antiperspirant containing ACH.

### Antiperspirant Effectiveness Data

| Number of Applications | Panelists | 48 hours | % Sweat Reduction |
|---|---|---|---|
| 4 | 30 (all females) | Essential Oil & Zinc Glycinate (EOZG3) | 50.7% |
| | | ACH Powder (D 1097) | 49.2% |

### Comparative Example 1 -Not Containing Non-Aluminum Metal as an Active Ingredient

The antiperspirant roll on formulation was manufactured using eucalyptus oil (1.5 w/w %) and menthe avernesis oil (1.5 w/w %). The entire list of ingredients for this formulation of Comparative Example is identified below in the table.

| Phase | Ingredients | % W/W |
|---|---|---|
| | | (EO3) |
| A | Water | 85.50 |
| | Acrylates/C10-30 Alkyl Acrylate Crosspolymer | 00.30 |
| | Sodium Hydroxide 20 % solution | 00.70 |
| B | Steareth-21 | 04.00 |
| | C12-15 alkyl benzoate | 03.00 |
| C | Eucalyptus globulus oil | 01.50 |
| | Mentha arvensis oil | 01.50 |
| | Propylene glycol | 03.00 |
| D | Phenoxyethanol | 00.50 |

First, acryaltes were dispersed in water to create a smooth dispersion. Then the dispersion was neutralized with sodium hydroxide to raise the pH to a range of 6.5 to 7.0. Once the clear gel was formed, the mixture was heated 70°C. In another vessel, phase B ingredients were mixed. At 70°C, the mixture of phase B ingredients was added into the clear gel with continuous stirring. At 55°C to 60°C, the phase C mixture was added to form an emulsion and the emulsion was homogenized for 8 to 10 minutes. Then at a temperature of 40°C - 45 °C , phenoxyethanol was added under stirring and the mixture was cooled down to room temperature. After 48 hours of production of the roll-on antiperspirant formulation containing only the essential oils as the antiperspirant active ingredients without any non-aluminum , quality and stability tests were performed as shown below:

### Quality parameters

| | |
|---|---|
| Appearance | White semi solid roll on |
| Color | Milky |
| Odor | Characteristics |
| pH 10% solution | 7.0-8.0 (7.6) |
| Viscosity - Brookfield Viscometer, Spindle | 4000-7000 cps (5800 cps) |
| No # 64 @ 20 rpm | |
| Thermal Stability @ 40°C ± 2°C / 75 % RH ± 5 % RH for 3 months | Stable |
| Centrifugal Test @ 40°C, 20 minutes @ 3000 rpm | Pass |

### Hot Room Study

The assessment of the antiperspirant effect was performed according to the FDA Guidelines for Effectiveness Testing of OTC Antiperspirant Drug Products. The methodology consisted of suspending any use of any product applied to underarm, beyond those supplied by Laboratory for a period of 17 days. After the conditioning period, it was carried out the initial assessment in sauna to check the amount of sweat produced per axilla without application of any products to obtain baseline sweat collection.

To be approved, it is necessary that the participant produces at least 100mg of sweating per axilla for two consecutive sessions (T1 and T2) of 20 minutes. The approved participants received four applications controlled of the investigational product of Comparative Example 1. The investigational product was applied to a 100 cm² area in the underarms. The investigational product application procedure was repeated four days consecutive. 0.4g ± 0.1 of the investigational products were applied in one of the armpits, previously randomized.

Approved participants received 4 applications controlled of the investigational product of Comparative Example 1 (ROLL ON EO-3) in one armpit and in the other, 4 applications controlled of the investigational product of Example 1 (ROLL ON EOZG-3). Posteriorly it was performed the sauna of 24 hours after the 4 controlled applications.

According to the study protocol and procedures used to evaluate the antiperspirant effect after four controlled application of the investigational products, the following is the conclusion:
- The investigational product Comparative Example 1 (ROLL ON EO-3) presented a reduction of 30.6% of sweating compared to the baseline condition.
- The investigational product Example 1 (ROLL ON EOZG-3) presented a reduction of 42.5% of sweating compared to the baseline condition.
- The difference in the results is about 12% between these two formulations.
- The P value obtained was <0.0001, indicating that there was a significant difference (P <0.005) between Example 1 (ROLL ON EOZG-3) and Comparative Example 1 (ROLL ON EO-3).
- It is also concluded that Comparative Example 1 (ROLL ON EO-3) is not comparable to ACH, the standard antiperspirant active.

### Antiperspirant Effectiveness Data

| Number of Applications | Panelists | 48 hours | % Sweat Reduction |
|---|---|---|---|
| 4 | 30 (all females) | Example 1 | 42.5% |
| | | (EOZG3) | |
| | | Comparative Example 1 (EO3) | 30.6% |

### Comparative Example 2 - Containing Collidal Silica Solution as an Antiperspirant Active Ingredient In Addition To Essential Oil & Non-Aluminum Metal

The antiperspirant roll on formulation was manufactured using eucalyptus oil (1.5 w/w %), menthe avernesis oil (1.5 w/w %) and colloidal silica 30% solution (7.0 w/w %, which is about 2% collidal silica) as the antiperspirant active ingredients. The entire list of ingredients for this formulation of Example 2 is identified below in the table.

| Ing. No. | Ingredients | EOCS % W/W |
|---|---|---|
| 1 | Water | 78.75 |
| 2 | Colloidal silica 30 % solution (∼ 2% Collidal silica) | 07.00 |
| 3 | Steareth-21 | 04.00 |
| 4 | C12-15 alkyl benzoate | 03.00 |
| 5 | Propylene glycol | 03.00 |
| 6 | Mentha arvensis oil | 01.50 |
| 7 | Eucalyptus globulus oil | 01.50 |
| 8 | Acrylates/c10-30 alkyl acrylate crosspolymer | 00.30 |
| 9 | Phenoxyethanol | 00.30 |
| 10 | Sodium hydroxide 20 % solution | 00.60 |
| 11 | Ethylenediaminetetraacetic Acid | 00.05 |

First, acrylates/c10-30 alkyl acrylate crosspolymer was slowly added into water under stirring and it was stirred for 30-40 minutes. After obtaining a lump free dispersion, sodium hydroxide 20% solution was added under stirring to make a gel formulation in a vessel. The gel was heated at 70°C. In another vessel, steareth-21 and c12-15 alkyl benzoate were mixed and melted and the mixture was heated to 70°C. At 70°C, the mixture of steareth-21 and c12-15 alkyl benzoate were added into the gel formulation. The mixture was homogenized at the temperature of between 60°C - 70°C and the roll-on base material was prepared. In a separate vessel, propylene glycol, mentha arvensis oil, eucalyptus globulus oil and ethylenediaminetetraacetic Acid were heated at 55-60°C. Then, this mixture was added to the prepared roll-on base material at 55-60°C. Then, phenoxyethanol was added at a temperature below 50°C and it was stirred well. Then, colloidal silica 30 % solution (∼ 2% collidal silica) was added at a temperature below 45°C under low stirring. The mixture was then cooled with stirring at normal room temperature to obtain a roll-on antiperspirant formulation. After 48 hours of production of the roll-on antiperspirant formulation, quality and stability tests were performed as shown below:

### Quality parameters

| **Parameters** | **EOCS** |
|---|---|
| Appearance | Air free liquid preparation |
| Color | Milky white |
| Odour | Pungent essential oils |
| pH 10 % solution | 8.5-9.5 |
| Viscosity cps (Brookfield viscometer, Sp No 64, rpm 20) | 100-1000cps |
| Centrifuge test (3000rpm, 20 minutes at 45C) | Pass, Stable |
| Stability test at 40°C, 3 months | Pass, Stable |

### Hot Room Study

The effectiveness of the antiperspirant active was tested by hot room test in accordance with the Guidelines of FDA. The test subjects (N=30, all female) undergone 17 days pf conditioning period, which consisted of suspending any use of any product applied to underarm, beyond those supplied by Laboratory for a period of 17 days. At the end of conditioning stage, baseline sweat collection was carried out. The hot room (or sauna) temperature was maintained at 37.7 ± 1°C and 35 ± 5% relative humidity. The products were applied at a ratio of 2 mg/cm² at a marked site of 100 cm² (10 x 10 cm) on each armpit using a disposable syringe. 24 hours after the last application (fourth application) of the products, the participants returned for another sauna session following the procedure described above. The collected pads were weighed and the difference between the initial and the final weight was calculated. The data was analyzed in order to compare both products.

According to the study protocol and procedures used to evaluate the antiperspirant effect after four controlled application of the investigational products, the following is the conclusion:
- Comparative Example 2 (EOCS3) reduced perspiration by 24% compared to the baseline condition.
- Example 1 (EOZG3) reduced perspiration by 40% compared to the baseline condition.
- The difference in the results is about 16% between these two formulations.
- There was statistically significant difference between the products Example 1 (EOZG3) and Comparative Example 2 (EOCS3) with p value of 0.03 (<0.05).
- The efficacy data indicate that colloidal silica does not have any antiperspirant effect.

### Antiperspirant Effectiveness Data

| Number of Applications | Panelists | 48 hours | % Sweat Reduction |
|---|---|---|---|
| 4 | 30 (all females) | Example 1 | 40% |
| | | (EOZG3) | |
| | | Comparative Example 2 (EOCS3) | 24% |

Although the invention herein has been described with reference to particular embodiments, it is to be understood that these embodiments are merely illustrative of the principles and applications of the present invention. It is therefore to be understood that numerous modifications may be made to the illustrative embodiments and that other arrangements may be devised without departing from the spirit and scope of the present invention as defined by the appended claims.

## Claims

1. An antiperspirant formulation comprising:
antiperspirant active ingredients,
a solvent, and
an additive,
wherein the antiperspirant active ingredients comprise an essential oil and a non-aluminum metal,
wherein the essential oil comprises mentha arvensis oil, eucalyptus globulus oil or a mixture thereof, and
the non-aluminum metal comprises zinc, calcium, magnesium or a mixture thereof.

2. The antiperspirant formulation of cliam 1, wherein the essential oil comprises the mixture of mentha arvensis oil and eucalyptus globulus oil.

3. The antiperspirant formulation of claim 1, wherein the antiperspirant active ingredients do not contain aluminum or aluminum-zirconium or zirconium.

4. The antiperspirant formulation of claim 1, wherein the antiperspirant active ingredients do not contain a colloidal silica solution.

5. The antiperspirant formulation of claim 1, wherein the essential oil is included in an amount of 0.5 to 10 % based on a total weight of the antiperspirant formulation.

6. The antiperspirant formulation of claim 1, wherein the non-aluminum metal is included in an amount of 0.1% to 10 % based on a total weight of the antiperspirant formulation.

7. The antiperspirant formulation of claim 1, wherein the non-aluminum metal comprises zinc, calcium, magnesium or a mixture thereof.

8. The antiperspirant formulation of claim 1, wherein a ratio of an amount of the essential oil to the non-metal aluminum metal is 0.1:1 to 10:1.

9. The antiperspirant formulation of claim 1, wherein the formulation is in a form of a stick, a powder, a liquid, a roll-on suspension, a lotion, an emulsion, a gel, a cream, a foam, an aerosol, a spray or a squeeze formulation.

10. A personal care composition comprising the antiperspirant formulation of claim 1 and a dermatologically or cosmetically acceptable carrier.

11. A personal care product comprising the personal care composition of claim 10, wherein the personal care product is a moisturizer, a cream, a lotion, a skin softener, a foundation, a night cream, a lipstick, a cleanser, a toner, a sunscreen, a cosmetic mask, a shampoo, a conditioner, an insect repellent, an anti-aging product, a body and hand preparation, a skin care preparation, a face and neck preparation or cosmetics.
